# EUROPEAN PATENT SPECIFICATION

(11) **EP 3 586 719 B1**
(45) Date of publication and mention of the grant of the patent: **01.07.2020**
(21) Application number: 19180902.9
(22) Date of filing: 18.06.2019
(51) Int. Cl.: A61B 1/06, A61B 1/00

(54) **ENDOSCOPE APPARATUS**
ENDOSKOPVORRICHTUNG
APPAREIL D'ENDOSCOPE

(30) Priority: 25.06.2018 JP 2018119764
(43) Date of publication of application: 01.01.2020
(73) Proprietor: Fujifilm Corporation, Minato-ku Tokyo 106-8620 (JP)
(72) Inventor: UTSUNOMIYA, Daisuke, Kanagawa 258-8538 (JP)
(74) Representative: Klunker IP Patentanwälte PartG mbB

(56) References cited:
- EP-A1- 2 462 860
- EP-A1- 3 366 191
- WO-A1-2017/057573
- WO-A1-2017/082047
- WO-A1-2018/038269
- WO-A1-2018/179991
- JP-A- 2004 321 244
- JP-A- 2016 067 780

## Description

### BACKGROUND OF THE INVENTION

### 1. Field of the Invention

The present invention relates to an endoscope apparatus that measures the size of a subject.

### 2. Description of the Related Art

A distance to an observation target object, the size of the observation target object, or the like is acquired in an endoscope apparatus. For example, in WO2017/199531A (corresponding to US2019/129037A1), of return light from an observation target, an observation image and return light of distance measuring light for distance measurement are branched by a branching optical system, and return light of the branched distance measuring light is received by a distance measuring light imaging element provided separately from an imaging element for the observation image. Based on the received return light of the distance measuring light, the distance to the observation target object is calculated by the time of flight method.

### SUMMARY OF THE INVENTION

In WO2017/199531A, the observation image obtained by the imaging element for observation does not include the component of the distance measuring light due to the use of the branching optical system, and therefore there is an advantage that the observation image is hardly affected by the distance measuring light. However, in addition to the branching optical system, by providing two imaging elements, there is a disadvantage that the whole endoscope apparatus including the endoscope is enlarged and expensive. Furthermore, in the clinic, there is a demand for space saving as well as medical cost saving. For this reason, it is necessary to suppress increase in size and cost of the whole endoscope apparatus.

Further relevant prior art documents relating to endoscopic imaging systems are WO 2018/038269, JP 2004321244, EP 2462860.

Further, it is difficult to completely branch only the distance measuring light in the branching optical system, and some part of the distance measuring light may enter the imaging element for the observation image in some cases. Since incidence of some of the distance measuring light affects the tint of the image of the observation image to a considerable degree, color reproducibility of the subject such as an observation image is impaired.

An object of the present invention is to provide an endoscope apparatus capable of presenting information on a subject such as the distance to an observation target object and the size of the observation target object without impairing the color reproducibility of the subject.

An endoscope apparatus according to the present invention comprises an illumination light source unit that emits illumination light to illuminate a subject, a specific light source unit that emits specific light, a light emission controller that performs a control of lighting of the illumination light and performs a control of repeatedly lighting and dimming of the specific light at a specific frame interval, an imaging element that images the subject, an imaging acquisition unit that acquires a first captured image obtained by imaging the subject illuminated with the illumination light and a second captured image obtained by imaging the subject illuminated with the illumination light and the specific light, a position specifying unit that specifies a position of a specific region to be obtained based on the specific light in the second captured image, based on the first captured image and the second captured image, an image processing unit that processes the first captured image or the second captured image to generate a specific image, based on the position of the specific region; and a display controller that causes a display unit to display the specific image.

It is preferable that the first captured image or the second captured image includes a noise component that interferes with the specifying of the position of the specific region by the position specifying unit, and the position specifying unit may include a noise component removing unit that removes the noise component from the second captured image to obtain a noise-removed second captured image based on the first captured image and the second captured image, and specifies the position of the specific region based on the noise-removed second captured image.

It is preferable that the noise component removing unit includes a color information conversion unit that converts the first captured image into a first color information image and converts the second captured image into a second color information image, a binarization processing unit that binarizes the first color information image into a binarized first color information image and binarizes the second color information image into a binarized second color information image, a mask image generating unit that obtains a mask image to remove the noise component from the second captured image based on the binarized first color information image and the binarized second color information image, and a removing unit that obtains the noise-removed second captured image based on the binarized second color information image and the mask image.

It is preferable that the image processing unit includes an image selecting unit that selects a processing target image which is a target to be processed from among the first captured image and the second captured image, and processes the image selected as the processing target image based on the position of the specific region. It is preferable that the image selecting unit selects the processing target image based on a state relating to the position of the specific region. It is preferable that the image selecting unit selects the processing target image according to an instruction from a user.

It is preferable that the specific light is auxiliary measurement light used to measure the subject, and the image processing unit generates the specific image in which a measurement marker that is set according to the position of the specific region is displayed in a superimposed manner on the first captured image or the second captured image. It is preferable that the measurement marker includes a first measurement marker indicating an actual size of the subject or a second measurement marker including a crossing line which is formed on the subject by the auxiliary measurement light and gradations serving as an index of a size of the subject on the crossing line. It is preferable that the type of the measurement marker is selectable by an instruction from a user. It is preferable that the specific light is excitation light to cause a fluorescent component included in the subject to be excited and emitted, and the image processing unit sets a fluorescent display region to display the fluorescent component according to the position of the specific region to generate the specific image that displays the fluorescent display region on the first captured image.

According to the present invention, it is possible to present information on a subject such as the distance to the observation target object and the size of the observation target object without impairing the color reproducibility of the subject.

### BRIEF DESCRIPTION OF THE DRAWINGS

Fig. 1 is a diagram showing the appearance of an endoscope apparatus.
Fig. 2 is a plan view showing a distal end portion of an endoscope.
Fig. 3 is a block diagram showing the function of the endoscope apparatus.
Fig. 4 is a block diagram showing an auxiliary measurement light emitting unit.
Fig. 5 is an explanatory diagram showing a spot SP formed on a subject by auxiliary measurement light.
Fig. 6 is an explanatory diagram showing a relationship between the distal end portion of the endoscope and a near end Px, an intermediate vicinity Py, and a far end Pz within a range Rx of an observation distance.
Fig. 7 is an explanatory diagram showing a light emission pattern and the like in a length measurement mode.
Fig. 8 is a block diagram showing functions of a signal processing unit.
Fig. 9 is a block diagram showing a flow of processing for obtaining a second captured image with noise removed.
Fig. 10 is an explanatory diagram showing a binarized first color information image.
Fig. 11 is an explanatory diagram showing a binarized second color information image.
Fig. 12 is an explanatory diagram showing a region of a noise component in the binarized first color information image.
Fig. 13 is an explanatory diagram showing a mask image.
Fig. 14 is an image diagram showing a spot display portion and a first measurement marker in a case where an observation distance corresponds to the near end Px.
Fig. 15 is an image diagram showing a spot display portion and a first measurement marker in a case where an observation distance corresponds to the intermediate vicinity Py.
Fig. 16 is an image diagram showing a spot display portion and a first measurement marker in a case where an observation distance corresponds to the far end Pz.
Fig. 17 is an explanatory diagram showing first measurement markers having a cruciform shape with gradations, a distorted cruciform shape, a circular-and-cruciform shape, and the shape of a measurement point group.
Fig. 18 is an explanatory diagram showing a graph paper-shaped chart that is used to measure a relationship between the position of a spot and the size of the first measurement marker in a case where an observation distance corresponds to the near end Px.
Fig. 19 is an explanatory diagram showing a graph paper-shaped chart that is used to measure a relationship between the position of a spot and the size of the first measurement marker in a case where an observation distance corresponds to the far end Pz.
Fig. 20 is a graph showing a relationship between the pixel position of a spot in an X direction and the number of pixels of the first measurement marker in the X direction.
Fig. 21 is a graph showing a relationship between the pixel position of a spot in a Y direction and the number of pixels of the first measurement marker in the X direction.
Fig. 22 is a graph showing a relationship between the pixel position of a spot in the X direction and the number of pixels of the first measurement marker in the Y direction.
Fig. 23 is a graph showing a relationship between the pixel position of a spot in the Y direction and the number of pixels of the first measurement marker in the Y direction.
Fig. 24 is an image diagram showing a marker that has the shape of three concentric circles having the same color.
Fig. 25 is an image diagram showing a marker that has the shape of three concentric circles having different colors.
Fig. 26 is an image diagram showing a marker having the shape of distorted concentric circles.
Fig. 27 is an image diagram showing a crossing line and gradations.
Fig. 28 is a block diagram showing a light source device comprising an excitation light source unit.
Fig. 29 is a block diagram showing a signal processing unit comprising a fluorescent display region setting unit.
Fig. 30 is an explanatory diagram showing a method of setting a fluorescent display region.

### DESCRIPTION OF THE PREFERRED EMBODIMENTS

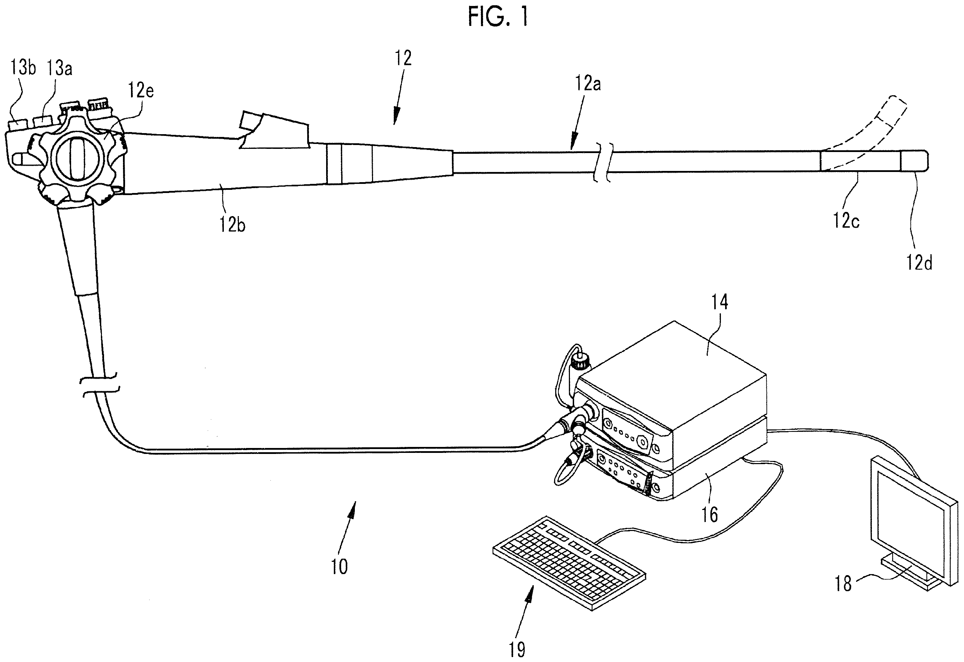

As shown in Fig. 1, an endoscope apparatus 10 includes an endoscope 12, a light source device 14, a processor device 16, a monitor 18, and a user interface 19. The endoscope 12 is optically connected to the light source device 14, and is electrically connected to the processor device 16. The processor device 16 is electrically connected to the monitor 18 (display unit) that displays an image. The user interface 19 is connected to the processor device 16, and is used for various setting operations and the like for the processor device 16. The user interface 19 includes a mouse and the like in addition to a keyboard shown in Fig. 1.

The endoscope 12 includes an insertion part 12a that is to be inserted into a subject, an operation part 12b that is provided at a proximal end portion of the insertion part 12a, and a bendable portion 12c and a distal end portion 12d that are provided at a distal end of the insertion part 12a. The bendable portion 12c operates to be bent by the operation of an angle knob 12e of the operation part 12b. The distal end portion 12d is oriented in a desired direction by the bending operation of the bendable portion 12c.

The endoscope 12 has a normal mode and a length measurement mode, and the two modes are switched by a mode changeover switch 13a that is provided on the operation part 12b of the endoscope 12. The normal mode is a mode for illuminating an observation target object with illumination light. In the length measurement mode, the observation target object is illuminated with the illumination light or the auxiliary measurement light and a measurement marker used for measuring the size of the observation target object or the like is displayed on the captured image obtained by the imaging of the observation target. The auxiliary measurement light is light used for measuring the subject.

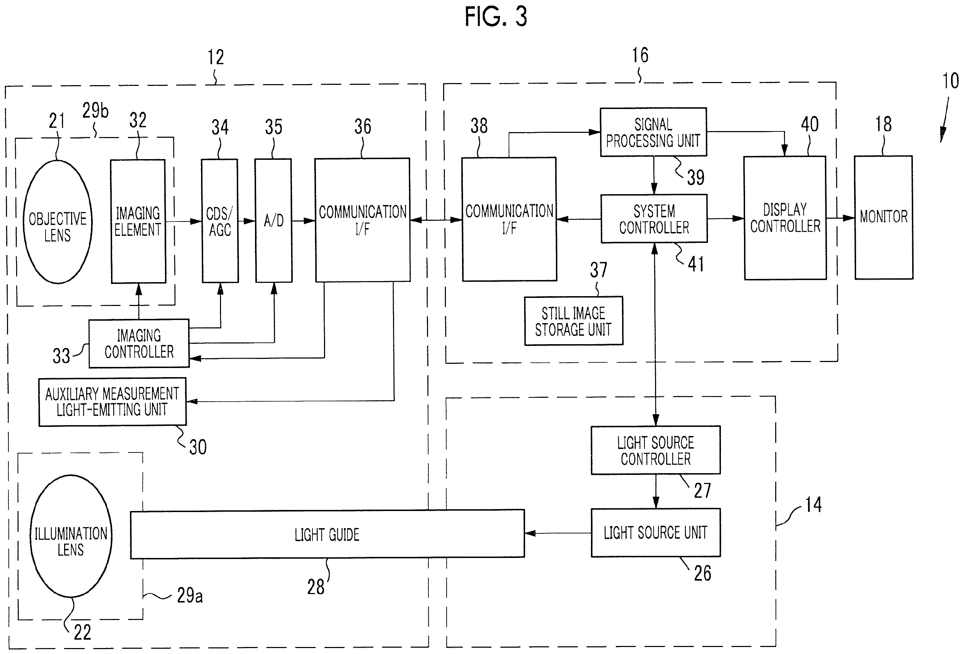

A freeze switch 13b (still image acquisition instructing unit) for operating a still image acquisition instruction instructing the acquisition of a still image of a captured image is provided on the operation part 12b of the endoscope 12. In a case where the user operates the freeze switch 13b, the screen of the monitor 18 is freeze-displayed, and an alert sound (for example, "pee") indicating that a still image is acquired is issued. The still image of the captured image obtained before and after the operation timing of the freeze switch 13b is stored in a still image storage unit 37 (see Fig. 3) in the processor device 16. Also, in a case where the length measurement mode is set, it is preferable to store measurement information, which will be described later, together with the still image of the captured image. The still image storage unit 37 is a storage unit such as a hard disk or a universal serial bus (USB) memory. In a case where the processor device 16 is connectable to a network, the still image of the captured image may be stored in a still image storage server (not shown) connected to the network instead of or in addition to the still image storage unit 37.

The still image acquisition instruction may be issued using an operation device other than the freeze switch 13b. For example, in a case where a foot pedal is connected to the processor device 16 and the user operates the foot pedal (not shown) with the foot, the still image acquisition instruction may be issued. Mode switching may be performed with a foot pedal. Further, a gesture recognition unit (not shown) for recognizing a user's gesture is connected to the processor device 16, and in a case where the gesture recognition unit recognizes a specific gesture performed by the user, the gesture recognition unit may instruct the processor device 16 to issue a still image acquisition instruction. The mode switching may also be performed using the gesture recognition unit.

Further, a line-of-sight input unit (not shown) provided near the monitor 18 is connected to the processor device 16, and in a case where the line-of-sight input unit recognizes that the user's line of sight is within a predetermined region in the monitor 18 for more than a certain period of time, the still image acquisition instruction may be issued. A voice recognition unit (not shown) may be connected to the processor device 16, and in a case where the voice recognition unit recognizes a specific voice uttered by the user, the still image acquisition instruction may be issued. The mode switching may also be performed using the voice recognition unit. An operation panel (not shown) such as a touch panel may be connected to the processor device 16, and in a case where the user performs a specific operation on the operation panel, a still image acquisition instruction may be issued. Mode switching may also be performed using the operation panel.

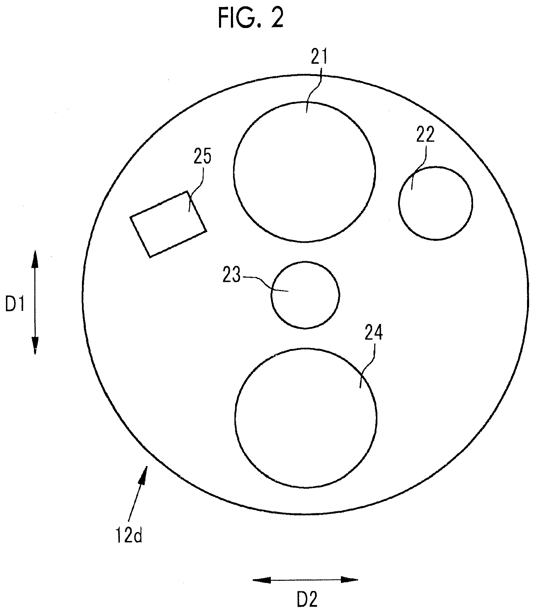

As shown in Fig. 2, the distal end portion of the endoscope 12 has a substantially circular shape; and is provided with an objective lens 21 that is positioned closest to a subject side among optical members of an imaging optical system of the endoscope 12, an illumination lens 22 that is used to irradiate a subject with illumination light, an auxiliary measurement lens 23 that is used to illuminate a subject with auxiliary measurement light to be described later, an opening 24 that allows a treatment tool to protrude toward a subject, and an air/water supply nozzle 25 that is used to supply air and water.

An optical axis Ax of the objective lens 21 extends in a direction perpendicular to the plane of paper. A vertical first direction D1 is orthogonal to the optical axis Ax, and a horizontal second direction D2 is orthogonal to the optical axis Ax and the first direction D1. The objective lens 21 and the auxiliary measurement lens 23 are arranged in the first direction D1.

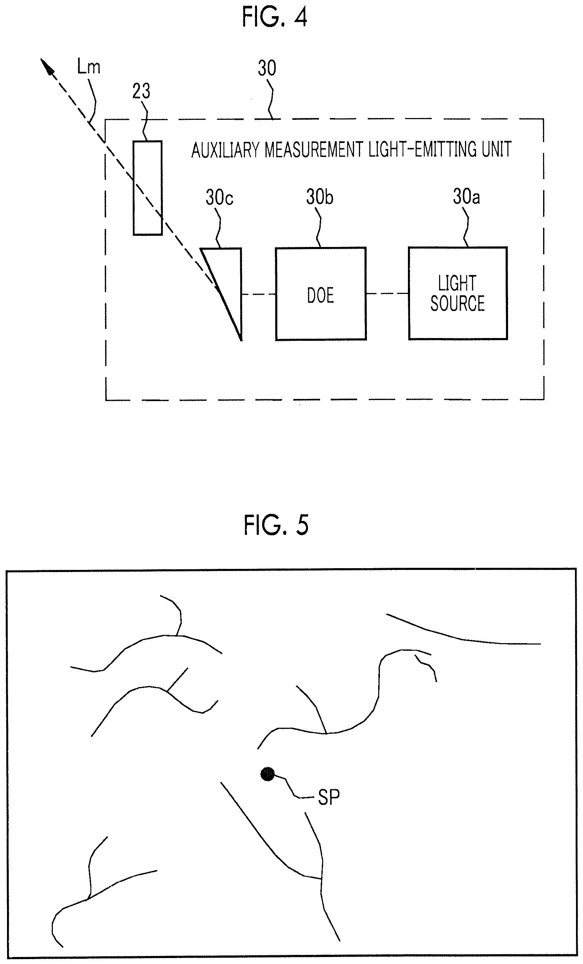

As shown in Fig. 3, the light source device 14 comprises a light source unit 26 and a light source controller 27. The light source unit 26 (illumination light source unit) generates illumination light for illuminating the subject. Illumination light emitted from the light source unit 26 is incident on a light guide 28, and is applied to a subject through the illumination lens 22. In the light source unit 26, a white light source emitting white light, a plurality of light sources, which include a white light source and a light source emitting another color light (for example, a blue light source emitting blue light), or the like is used as a light source of illumination light. The light source controller 27 is connected to a system controller 41 of the processor device 16. The light source controller 27 controls the light source unit 26 based on a command output from the system controller 41. The system controller 41 (light emission controller) also controls the light source 30a (see Fig. 4) of an auxiliary measurement light-emitting unit 30 in addition to giving an instruction on the light source control to the light source controller 27. Details of the light source control by the system controller 41 will be described later.

The distal end portion 12d of the endoscope 12 is provided with an illumination optical system 29a, an imaging optical system 29b, and the auxiliary measurement light-emitting unit 30. The illumination optical system 29a includes the illumination lens 22, and an observation target object is irradiated with light, which is emitted from the light guide 28, through the illumination lens 22. The imaging optical system 29b includes the objective lens 21 and an imaging element 32. Light reflected from the observation target object is incident on the imaging element 32 through the objective lens 21. Accordingly, the reflected image of the observation target object is formed on the imaging element 32.

The imaging element 32 is a color imaging sensor, and captures the reflected image of a subject to output image signals. It is preferable that the imaging element 32 is a charge coupled device (CCD) imaging sensor, a complementary metal-oxide semiconductor (CMOS) imaging sensor, or the like. The imaging element 32 used in the invention is a color imaging sensor that is used to obtain RGB image signals corresponding to three colors of R (red), G (green), and B (blue). The imaging element 32 is controlled by an imaging controller 33.

The image signals output through the imaging element 32 are transmitted to a CDS/AGC circuit 34. The CDS/AGC circuit 34 performs correlated double sampling (CDS) or auto gain control (AGC) on the image signals that are analog signals. The image signals, which have been transmitted through the CDS/AGC circuit 34, are converted into digital image signals by an analog/digital converter (A/D converter) 35. The digital image signals, which have been subjected to A/D conversion, are input to the processor device 16 through a communication interface (I/F) 36.

As shown in Fig. 3, the processor device 16 comprises a communication interface (I/F) 38 that is connected to the communication I/F 36 of the endoscope 12, a signal processing unit 39, a display controller 40, and a system controller 41. The communication I/F receives the image signals, which are transmitted from the communication I/F 36 of the endoscope 12, and transmits the image signals to the signal processing unit 39. A memory, which temporarily stores the image signals received from the communication I/F 38, is built in the signal processing unit 39, and the signal processing unit 39 processes an image signal group, which is a set of the image signals stored in the memory, to generate a captured image. In a case where the length measurement mode is set, the signal processing unit 39 may execute a structure emphasis processing for emphasizing a structure of a blood vessel or the like on a captured image, or a color difference emphasis processing for expanding the color difference between the normal part and the diseased part in the observation target.

The display controller 40 causes the monitor 18 to display the captured image, which is generated by the signal processing unit 39. The system controller 41 controls the imaging element 32 through the imaging controller 33 that is provided in the endoscope 12. The imaging controller 33 also controls the CDS/AGC circuit 34 and the A/D converter 35 according to the control of the imaging element 32.

As shown in Fig. 4, the auxiliary measurement light-emitting unit 30 (specific light source unit) comprises a light source 30a, a diffractive optical element (DOE) 30b, a prism 30c, and the auxiliary measurement lens 23. The light source 30a is to emit light having a color that can be detected by pixels of the imaging element 32 (specifically visible light), and includes a light-emitting element, such as a laser diode (LD) that is a laser light source or a light-emitting diode (LED), and a condenser lens that condenses light emitted from the light-emitting element.

It is preferable that the wavelength of the light emitted from the light source 30a is, for example, red light of 600 nm or more and 650 nm or less. Alternatively, green light of 495 nm or more and 570 nm or less may be used. The light source 30a is controlled by the system controller 41, and emits light based on a command output from the system controller 41. The DOE 30b converts light emitted from the light source into auxiliary measurement light (specific light) for obtaining measurement information.

The prism 30c is an optical member that is used to change the travel direction of the auxiliary measurement light converted by the DOE 30b. The prism 30c changes the travel direction of the auxiliary measurement light such that the auxiliary measurement light intersects with the field of view of the imaging optical system including the objective lens 21 and lens groups. The details of the traveling direction of the auxiliary measurement light will also be described later. A subject is irradiated with auxiliary measurement light, which is emitted from the prism 30c, through the auxiliary measurement lens 23. By irradiating the subject with the auxiliary measurement light, as shown in Fig. 5, a spot SP as a circular region is formed in the subject. The position of the spot SP is characterized by the position specifying unit, and a measurement marker indicating the actual size is set according to the position of the spot SP. The set measurement marker is displayed on the captured image. As described later, the measurement marker includes a plurality of types of measurement markers, such as a first measurement marker and a second measurement marker, and selection as to which type of measurement marker is to be displayed on the captured image can be made by the user's instruction. For example, the user interface 19 is used for an instruction of the user.

An auxiliary measurement slit may be formed at the distal end portion 12d of the endoscope, instead of the auxiliary measurement lens 23. Further, it is preferable to apply an anti-reflection coating (AR coating) (anti-reflection section) to the auxiliary measurement lens 23. The reason why the anti-reflection coating is provided as described above is that, in a case where the auxiliary measurement light is reflected without passing through the auxiliary measurement lens 23 and the proportion of the auxiliary measurement light applied to the subject decreases, a position specifying unit 50, which will be described later, is difficult to recognize the position of the spot SP formed on the subject by the auxiliary measurement light.

The auxiliary measurement light-emitting unit 30 may be anything as long as it can emit auxiliary measurement light toward the field of view of the imaging optical system. For example, the light source 30a may be provided in the light source device and light emitted from the light source 30a may be guided to the DOE 30b by optical fibers. Further, the prism 30c may not be used and the directions of the light source 30a and the DOE 30b may be inclined with respect to the optical axis Ax such that the auxiliary measurement light is emitted in a direction crossing the field of view of the imaging optical system.

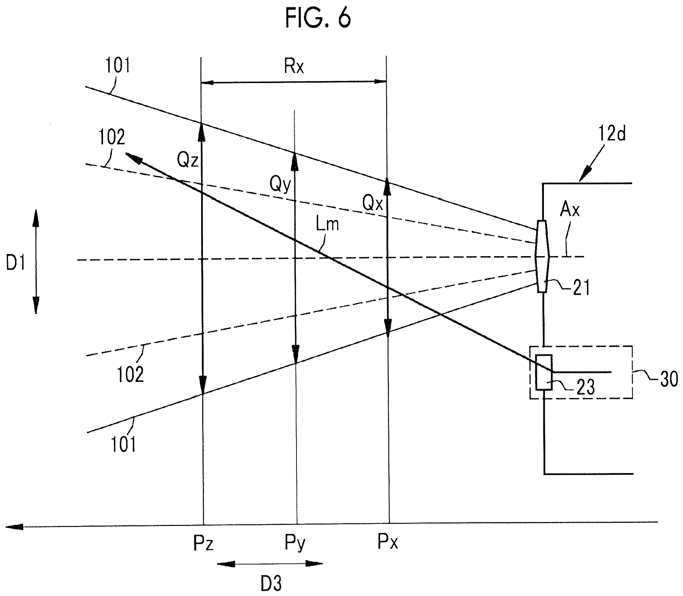

As shown in Fig. 6, with respect to the traveling direction of the auxiliary measurement light, the auxiliary measurement light is emitted in a state where an optical axis Lm of the auxiliary measurement light crosses the optical axis Ax of the objective lens 21. In a case where a subject can be observed in a range Rx of an observation distance, it is understood that the positions (points where the respective arrows Qx, Qy, and Qz cross the optical axis Ax) of the spot SP formed on the subject by the auxiliary measurement light in imaging ranges (indicated by arrows Qx, Qy, and Qz) at a near end Px, an intermediate vicinity Py, and a far end Pz of the range Rx are different from each other. The imaging angle of view of the imaging optical system is represented by a region between two solid lines 101, and measurement is performed in a central region (a region between two dotted lines 102), in which an aberration is small, of this imaging angle of view.

As described above, by emitting the auxiliary measurement light in a state where the optical axis Lm of the auxiliary measurement light intersects with the optical axis Ax, the sensitivity of the movement of the spot position with respect to the change of the observation distance is high, and therefore, the size of the subject can be measured with high accuracy. Then, by imaging the subject illuminated with auxiliary measurement light with the imaging element 32, the captured image including the spot SP is obtained. In the captured image, the position of a spot SP depends on a relationship between the optical axis Ax of the objective lens 21 and the optical axis Lm of auxiliary measurement light and an observation distance. However, the shorter the observation distance is, the higher the number of pixels showing the same actual size (for example, 5 mm) is, and the longer the observation distance is, the lower the number of pixels showing the same actual size.

Accordingly, as described in detail later, by storing information indicating the relationship between the position of the spot SP and the measurement information (the number of pixels) corresponding to the actual size of the subject in advance, the measurement information can be calculated from the position of the spot SP.

The details of the light source control by the system controller 41 will be described. In a case where the normal mode is set, the system controller 41 instructs the light source unit 26 of the light source device 14 to constantly emit the illumination light. As a result, illumination light is emitted from the light source unit 26. The subject is irradiated with the illumination light through the light guide 28. In the normal mode, the light source 30a of the auxiliary measurement light-emitting unit 30 is stopped.

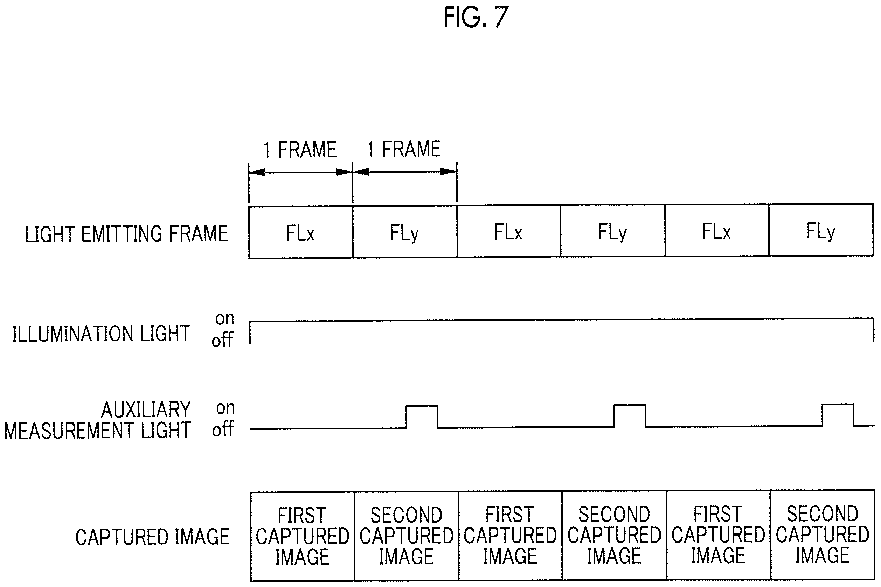

On the other hand, in a case where the length measurement mode is set, the system controller 41 controls the light source unit 26 of the light source device 14 so as to continuously emit the illumination light, and controls the light source 30a of the auxiliary measurement light-emitting unit 30 so that the auxiliary measurement light is repeatedly lightened and dimmed at a specific frame interval. Specifically, as shown in Fig. 7, in a case where the specific frame interval is set to one frame interval, the light emission frame FLx, in which only the illumination light is lightened (on) and the auxiliary measurement light is dimmed (off), and the light emitting frame FLy, in which both the illumination light and the auxiliary measurement light are lightened (on), are alternately performed. In the case of the light emitting frame FLx, a first captured image is obtained as a captured image obtained by imaging the subject illuminated with the illumination light by the imaging element 32. In the case of the light emitting frame FLy, a second captured image is obtained as a captured image obtained by imaging the subject illuminated with the illumination light and the auxiliary measurement light by the imaging element 32. Incidentally, in the light emitting frame FLx, instead of completely dimming the auxiliary measurement light, the light intensity of the auxiliary measurement light can be reduced to a specific value by being compared with the light emitting frame FLy. Although the captured image is an RGB image of three colors, other color images (a brightness signal Y and color difference signals Cr, Cb) may be used.

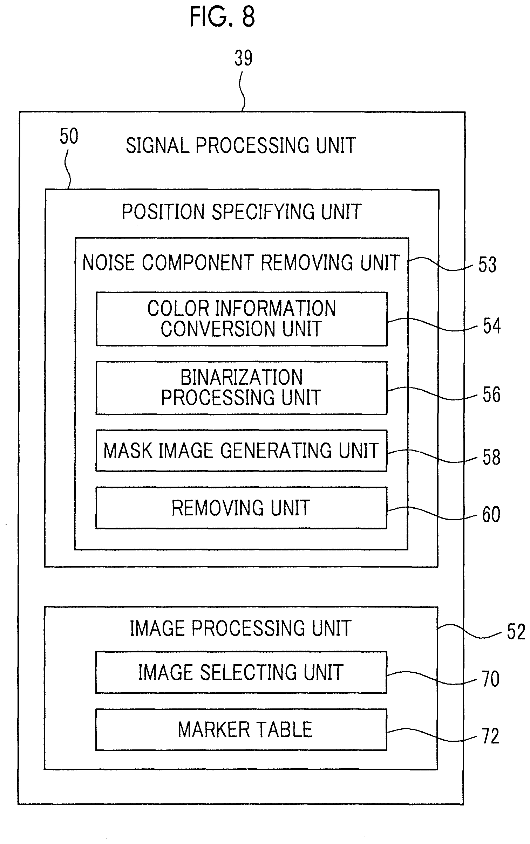

As shown in Fig. 8, the signal processing unit 39 of the processor device 16 comprises the position specifying unit 50 that specifies the position of the spot SP in the second captured image in order to perform the position recognition of the spot SP (specific region) and the setting of the measurement marker, and an image processing unit 52 that processes the first captured image or the second captured image to generate a specific image based on the position of the spot SP. The specific image is displayed on the monitor 18 by the display controller 40. In a case where the length measurement mode is set, the first captured image and the second captured image are input to the signal processing unit 39 as captured images. The first captured image or the second captured image is acquired by the communication I/F 38 (imaging acquisition unit).

The position specifying unit 50 comprises a noise component removing unit 53 that removes a noise component that interferes with the specifying of the position of the spot SP. In the second captured image, in the case where a color is included which is different from the color of the auxiliary measurement light forming the spot SP, but is close to the auxiliary measurement light (color approximate to auxiliary measurement light), the position of the spot SP may not be accurately specified in some cases. Therefore, the noise component removing unit 53 removes the component of the color approximate to auxiliary measurement light from the second captured image, as a noise component. The position specifying unit 50 specifies the position of the spot SP based on the noise-removed second captured image from which the noise component has been removed.

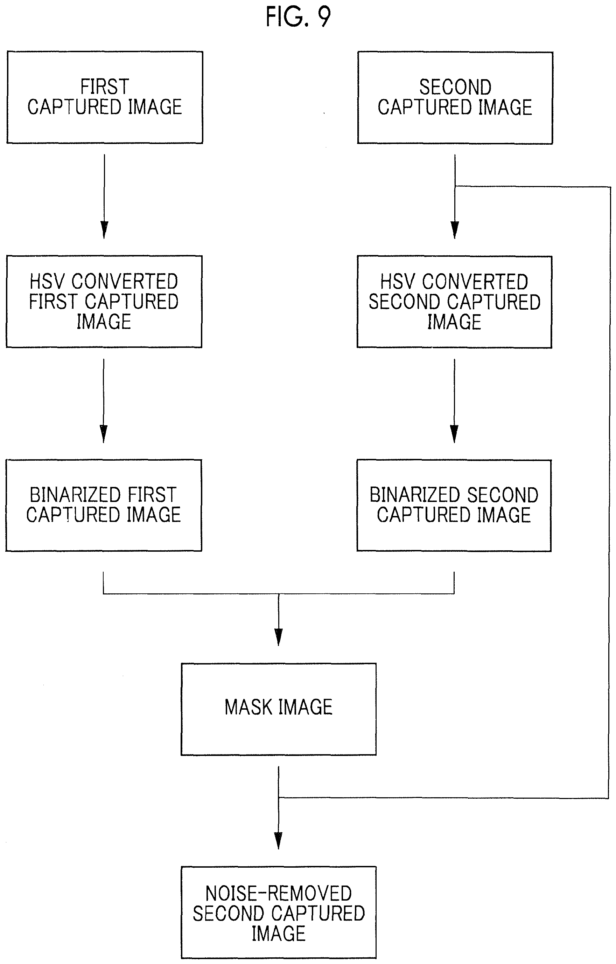

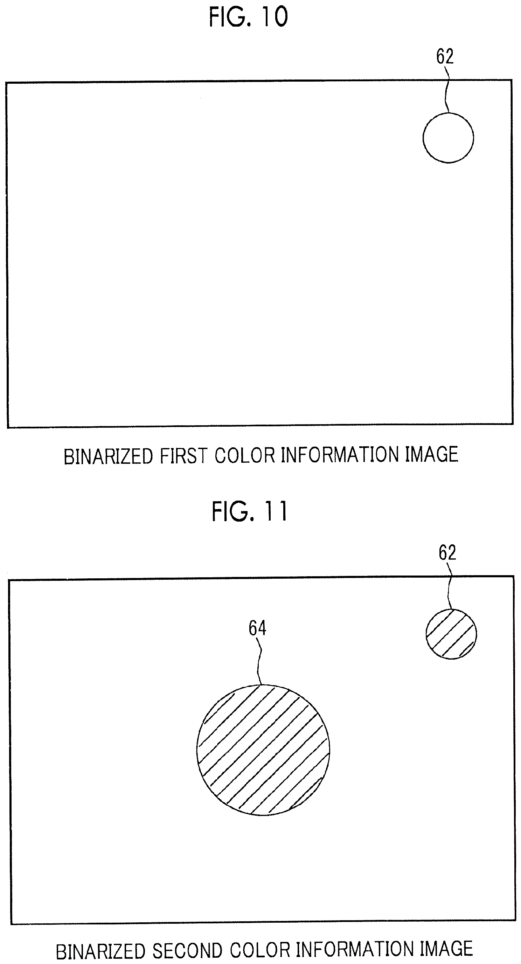

The noise component removing unit 53 comprises a color information conversion unit 54, a binarization processing unit 56, a mask image generating unit 58, and a removing unit 60. A process flow for obtaining the noise-removed second captured image will be described with reference to Fig. 9. The color information conversion unit 54 converts the first captured image which is an RGB image into a first color information image and converts the second captured image which is an RGB image into a second color information image. For example, it is preferable to set HSV (H (hue), S (saturation), V (value)) for the color information. Besides, color difference Cr, Cb may be used for the color information. The binarization processing unit 56 binarizes the first color information image into a binarized first color information image and binarizes the second color information image into a binarized second color information image. For the threshold value for binarization, a binarization threshold including the color of auxiliary measurement light is used. As shown in Fig. 10 and Fig. 11, the binarized first color information image includes color information 62 of the noise component. The binarized second color information image includes the color information 62 of the noise component in addition to the color information 64 of the auxiliary measurement light.

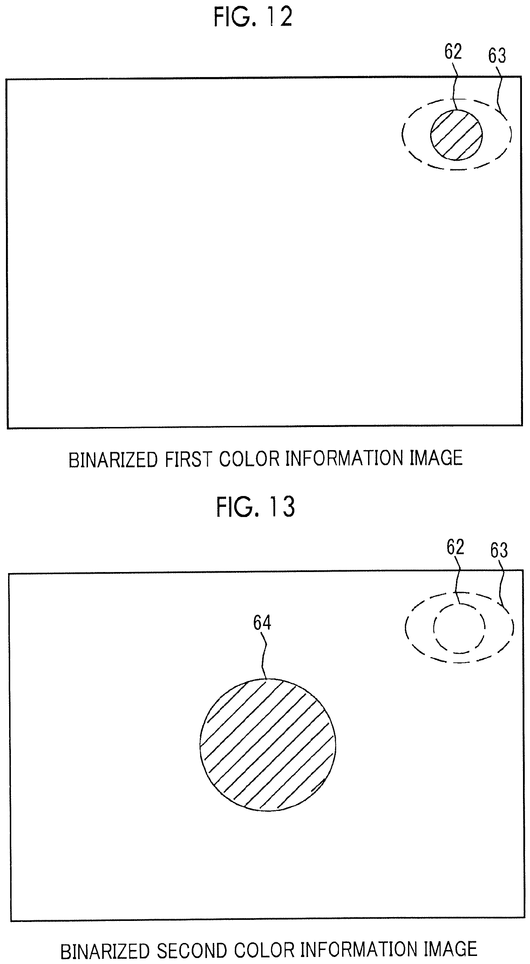

Based on the binarized first color information image and the binarized second color information image, the mask image generating unit 58 removes noise component color information from the second captured image, and generates a mask image for extracting color information of the auxiliary measurement light. As shown in Fig. 12, the mask image generating unit 58 specifies a noise component region 63 having a noise component from the noise component included in the binarized first captured image. Then, as shown in Fig. 13, the mask image generating unit 58 generates a mask image that sets an extraction region in which color information regarding the region of the color information 64 of the auxiliary measurement light is extracted, and a non-extraction region in which color information regarding the noise component region is not extracted, from the binarized second color information image. It is preferable that the noise component region 63 is larger than the region occupied by the color information 62 of the noise component. This is because, in the case where camera shake or the like occurs, the region of the color information 62 of the noise component becomes larger than in the case where there is no camera shake or the like. Figs. 10 to 13 schematically show the binarized first color information image, the binarized second color information image, the region of the noise component, and the mask image for the purpose of description.

The removing unit 60 extracts color information from the second color information image using the mask image, thereby obtaining the noise-removed second color information image in which the color information of the noise component is removed and the color information of the auxiliary measurement light is extracted. The noise-removed second color information image becomes the noise-removed second captured image by performing RGB conversion processing of returning color information to an RGB image. The position specifying unit 50 specifies the position of the spot SP based on the noise-removed second captured image. Since the noise component is removed from the noise-removed second captured image, the position of the spot SP can be accurately specified.

The image processing unit 52 has an image selecting unit 70 and a marker table. The image selecting unit 70 selects a processing target image which is a target image to be processed based on the position of the spot SP, among the first captured image or the second captured image. The image processing unit 52 performs processing on the image selected as the processing target image, based on the position of the spot SP. The image selecting unit 70 selects the processing target image based on the state regarding the position of the spot SP. The image selecting unit 70 may select an image to be processed according to an instruction from the user. For example, the user interface 19 is used for an instruction by the user.

Specifically, in a case where the spot SP is within a specific range during a specific period, it is considered that there is little movement of the subject or the distal end portion 12d of the endoscope, and accordingly, the first captured image is selected as a target image to be processed. In a case where there is little movement described above, it is considered that, even though there is no spot SP, easy alignment with the lesion included in the subject can be achieved. In addition, since the first captured image does not include the color component of the auxiliary measurement light, color reproducibility of the subject is not impaired. On the other hand, in a case where the position of the spot SP is not within the specific range during the specific period, it is considered that there is much movement of the subject or the distal end portion 12d of the endoscope, and accordingly the second captured image is selected as a target image to be processed. In a case where the movement is large described above, the user operates the endoscope 12 such that the spot SP is located in the lesion. This facilitates alignment with the lesion.

The image processing unit 52 generates a first measurement marker indicating the actual size of the subject as a measurement marker based on the position of the spot SP in the second captured image. The image processing unit 52 calculates the size of a marker from the position of the spot with reference to a marker table 72 where a relationship between the position of the spot SP in the second captured image and the first measurement marker representing the actual size of the subject is stored. Then, the image processing unit 52 generates the first measurement marker corresponding to the size of the marker.

After the specifying of the position of the spot and the generating of the first measurement marker are completed, a display controller 40 allows a monitor 18 to display a spot display portion and the first measurement marker at the position of the spot in a first captured image (where the spot SP does not appear), which is obtained through the imaging of the subject illuminated with illumination light. In diagnosis by the actual endoscope using the length measurement mode, the user inserts or removes the endoscope 12 into the body of a patient until the distal end portion 12d of the endoscope reaches the affected region of the patient. At the time when the distal end portion 12d of the endoscope reaches the affected part, the auxiliary measurement light or the measurement marker is aligned with the affected part. The size of the affected part is measured using the measurement marker. According to the result of the diagnosis based on the size of the affected part, the policy regarding treatment (excision, or the like) concerning the affected part is determined.

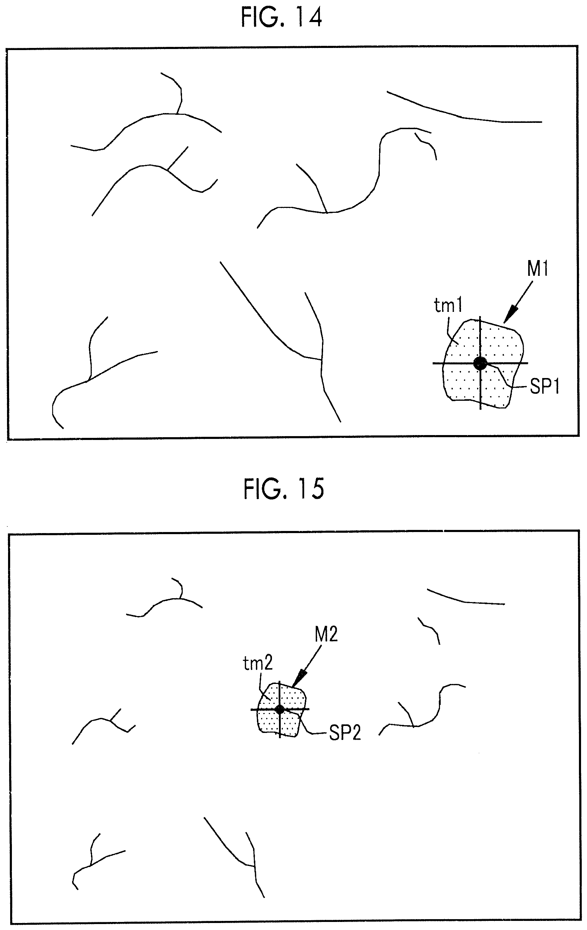

As the first measurement marker, for example, a cruciform measurement marker is used. As shown in Fig. 14, a cruciform marker M1, which represents the actual size of 5 mm (a horizontal direction and a vertical direction of the second captured image), is displayed at the center of a spot display portion SP1 formed on a tumor tm1 of a subject in a case where an observation distance is close to the near end Px. Since the tumor tm1 and a range, which is determined by the cruciform marker M1, substantially match each other, the size of the tumor tm1 can be measured as about 5 mm. In the first captured image, the spot display portion may not be displayed and only the first measurement marker may be displayed.

Since a spot formed by auxiliary measurement light does not appear in the first captured image, the spot display portion is displayed at a portion, which corresponds to the position of the recognized spot SP, with brightness and a color that allow a user to know the position of the spot. In a case where the spot and a portion of the subject to be observed have the same color (red color), the visibility of the portion to be observed may deteriorate due to the spread of the color. However, since a spot display portion representing the spot is displayed in the first captured image where the spot does not appear as described above, the bleeding of the color caused by auxiliary measurement light can be avoided. Accordingly, the visibility of the portion to be observed does not deteriorate.

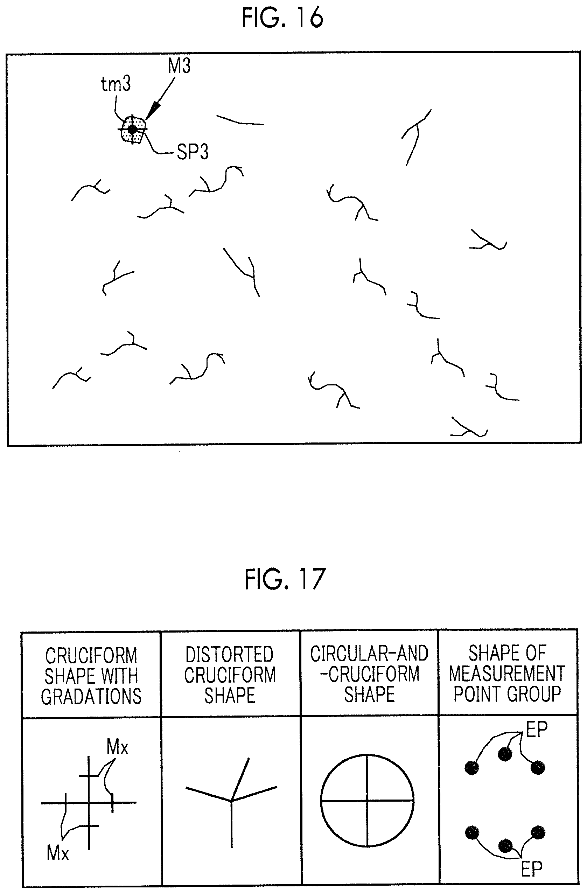

Similarly, as shown in Fig. 15, a cruciform marker M2, which represents the actual size of 5 mm (a horizontal direction and a vertical direction of the second captured image), is displayed at the center of a spot display portion SP2 formed on a tumor tm2 of a subject in a case where an observation distance is close to the intermediate vicinity Py. Further, as shown in Fig. 16, a cruciform marker M3, which represents the actual size of 5 mm (a horizontal direction and a vertical direction of the second captured image), is displayed at the center of a spot display portion SP3 formed on a tumor tm3 of a subject. As described above, the position of a spot on the imaging surface of the imaging element 32 varies according to an observation distance. For this reason, the display position of the marker also varies. As shown in Figs. 14 to 16, the size of the first measurement marker corresponding to the same actual size of 5 mm is reduced as an observation distance is increased.

The spot and the marker are displayed in Figs. 14 to 16 such that the center of the spot SP and the center of the marker match each other, but the first measurement marker may be displayed at a position apart from the spot SP in a case where a problem in terms of measurement accuracy does not occur. Even in this case, it is preferable that the first measurement marker is displayed near the spot. Further, a deformed first measurement marker is not displayed, and the distortion of a captured image may be corrected and an undeformed first measurement marker may be displayed in a corrected captured image.

Furthermore, the first measurement marker corresponding to the actual size of a subject of 5 mm is displayed in Figs. 14 to 16, but the actual size of a subject may be set to any value

(for example, 2 mm, 3 mm, 10 mm, or the like) according to an observation target object or the purpose of observation. Furthermore, in Figs. 14 to 16, the first measurement marker has a cruciform shape where a vertical line and a horizontal line are orthogonal to each other. However, as shown in Fig. 17, the first measurement marker may have a cruciform shape with gradations where gradations Mx are given to at least one of a vertical line or a horizontal line of a cruciform shape. Furthermore, the first measurement marker may have a distorted cruciform shape of which at least one of a vertical line or a horizontal line is inclined. Furthermore, the first measurement marker may have a circular-and-cruciform shape where a cruciform shape and a circle are combined with each other. In addition, the first measurement marker may have the shape of a measurement point group where a plurality of measurement points EP corresponding to an actual size from a spot display portion are combined with each other. Furthermore, one first measurement marker may be displayed or a plurality of first measurement markers may be displayed, and the color of the first measurement marker may be changed according to an actual size.

A method of making the marker table 72 will be described below. A relationship between the position of a spot and the size of a marker can be obtained through the imaging of a chart where a pattern having the actual size is regularly formed. For example, spot-like auxiliary measurement light is emitted to the chart, a graph paper-shaped chart including lines (5 mm) having the same size as the actual size or lines (for example, 1 mm) having a size smaller than the actual size is imaged while an observation distance is changed to change the position of a spot, and a relationship between the position of a spot (pixel coordinates of the spot on the imaging surface of the imaging element 32) and the number of pixels corresponding to the actual size (pixels showing 5 mm that is the actual size) is acquired.

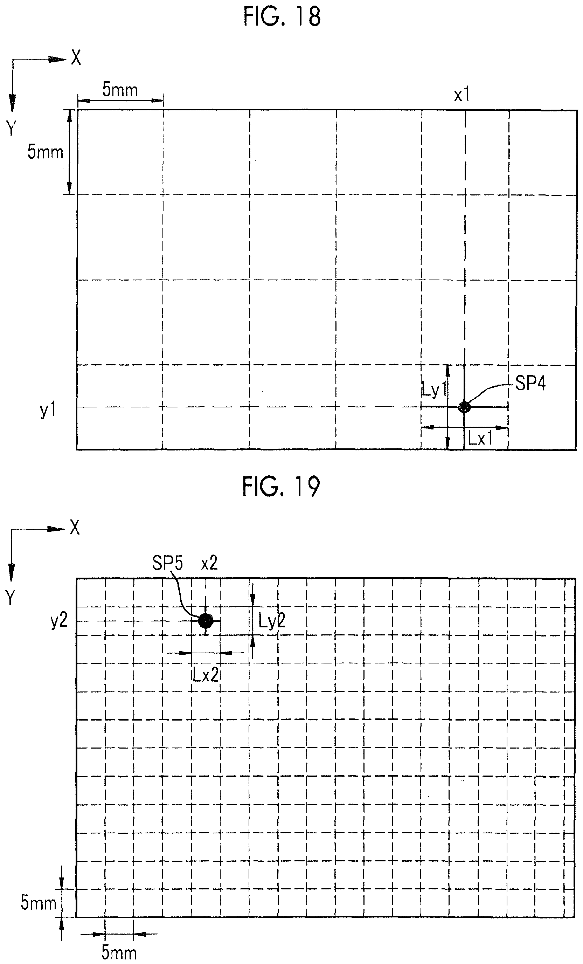

As shown in Fig. 18, (x1,y1) means the pixel position of a spot SP4 in an X direction and a Y direction on the imaging surface of the imaging element 32 (an upper left point is the origin of a coordinate system). The number of pixels in the X direction, which corresponds to the actual size of 5 mm, at the position (x1,y1) of the spot SP4 is denoted by Lx1, and the number of pixels in the Y direction is denoted by Ly1. This measurement is repeated while an observation distance is changed. Fig. 19 shows a state where the chart including lines having a size of 5 mm as in Fig. 18 is imaged, but an interval between the lines is narrow since this state is a state where an observation distance is closer to the far end than that in the state of Fig. 18. In the state of Fig. 19, the number of pixels in the X direction, which corresponds to the actual size of 5 mm, at the position (x2, y2) of a spot SP5 on the imaging surface of the imaging element 32 is denoted by Lx2, and the number of pixels in the Y direction is denoted by Ly2. Then, while an observation distance is changed, the same measurement as those in Figs. 18 and 19 is repeated and the results thereof are plotted. The charts are shown in Figs. 18 and 19 without consideration for the distortion of the objective lens 21.

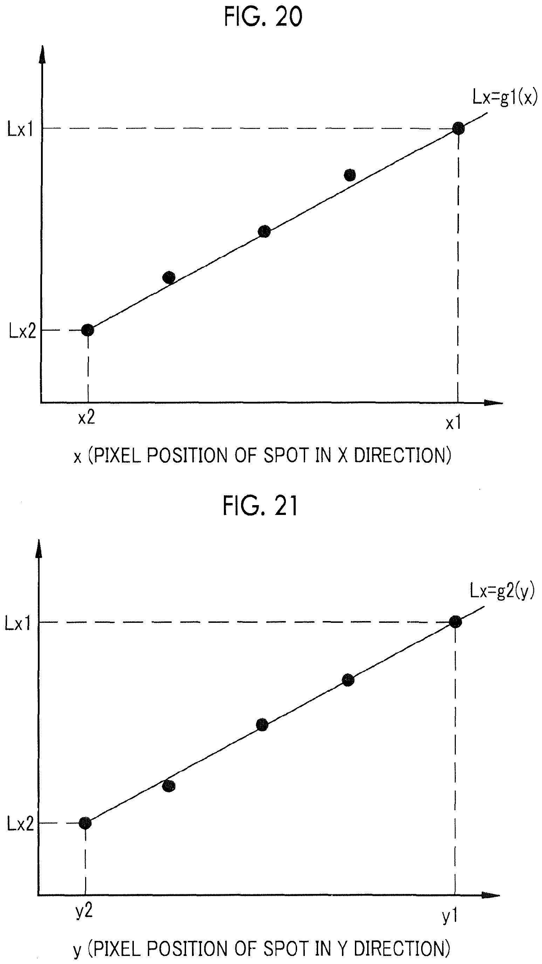

Fig. 20 shows the relationship between the X coordinate of the position of the spot and Lx (the number of pixels in the X direction), and Fig 21 shows the relationship between the Y coordinate of the spot position and Lx. Lx is expressed by "Lx=g1(x)" as a function of the position in the X direction from the relationship of Fig. 20, and Lx is expressed by "Lx=g2(y)" as a function of the position in the Y direction from the relationship of Fig. 21. The functions g1 and g2 are obtained from the above-mentioned plotted results by, for example, a least-square method.

The X-coordinate of a spot corresponds to the Y-coordinate of a spot one to one, and basically the same results are obtained (the same number of pixels is obtained at the position of the same spot) even though any one of the function g1 or g2 is used. Accordingly, in a case where the size of the first measurement marker is to be calculated, any one of the function g1 or g2 may be used and a function of which sensitivity to a change in the number of pixels with respect to a change in position is higher may be selected from the functions g1 and g2. In a case where the values of the functions g1 and g2 are significantly different from each other, it may be determined that "the position of a spot cannot be recognized".

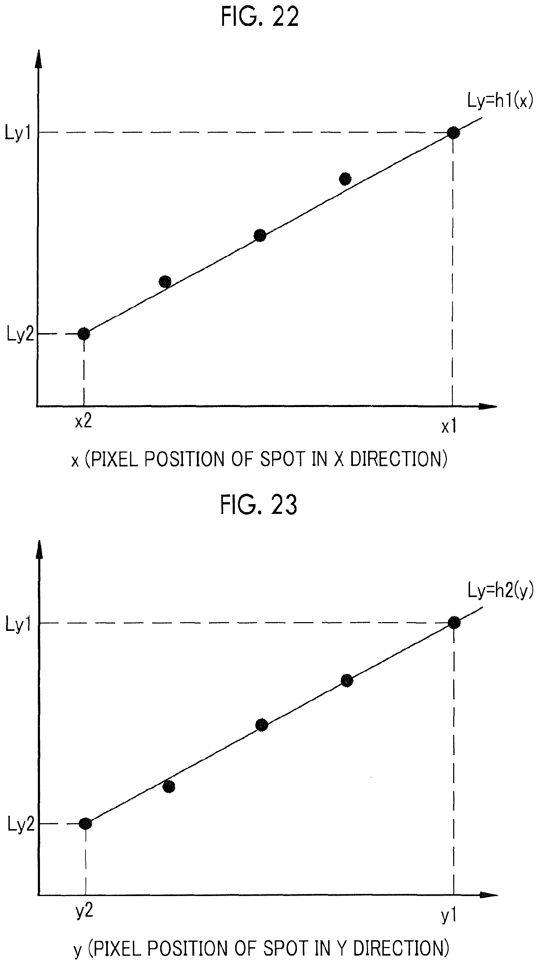

Fig. 22 shows a relationship between the X-coordinate of the position of a spot and Ly (the number of pixels in the Y direction), and Fig. 23 shows a relationship between the Y-coordinate of the position of a spot and Ly. Ly is expressed by "Ly=h1(x)" as the coordinate of the position in the X direction from the relationship of Fig. 22, and Ly is expressed by "Ly=h2(y)" as the coordinate of the position in the Y direction from the relationship of Fig. 23. Any one of the function h1 or h2 may also be used as Ly as in the case of Lx.

The functions g1 , g2, h1, and h2 obtained as described above are stored in a marker table in the form of a look-up table. The functions g1 and g2 may be stored in a marker table in the form of a function.

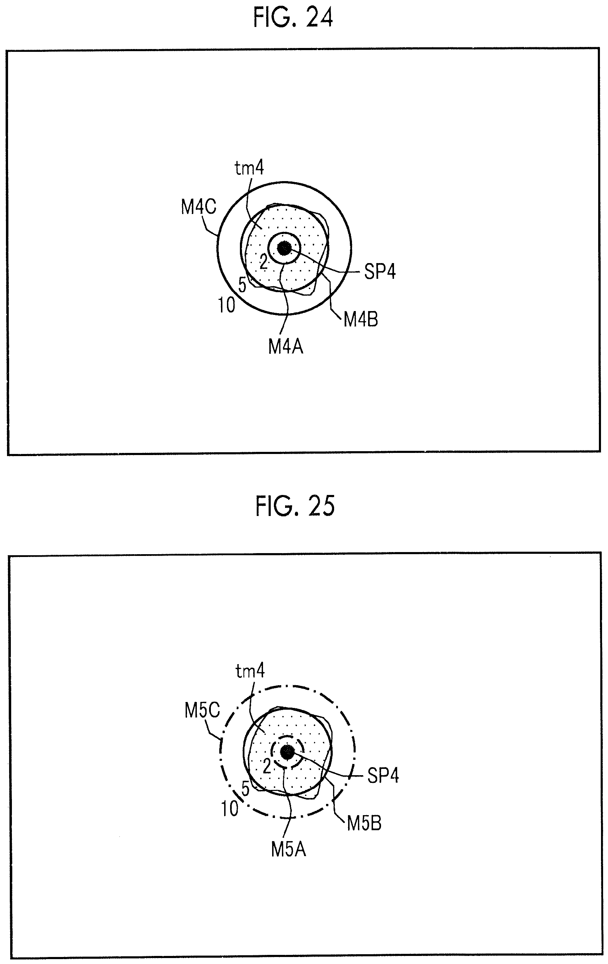

In the second embodiment, as shown in Fig. 24, three concentric circular markers M4A, M4B, and M4C having different sizes (of which diameters are 2 mm, 5 mm, and 10 mm, respectively) may be displayed around a spot display portion SP4 formed on a tumor tm4 in the first captured image as the first measurement marker. In the case of the three concentric circular markers, it is possible to save a trouble of switching a marker since the plurality of markers are displayed, and it is possible to perform measurement even in a case where a subject has a non-linear shape. In a case where a plurality of concentric circular markers are displayed around a spot, a size and a color are not designated for each marker but combinations of a plurality of conditions may be prepared in advance and one can be selected from these combinations.

In Fig. 24, all the three concentric circular markers are displayed with the same color (black). However, in a case where a plurality of concentric circular markers are to be displayed, a plurality of color concentric circular markers of which colors are different from each other may be used. As shown in Fig. 25, a marker M5A is displayed by a dotted line representing a red color, a marker M5B is displayed by a solid line representing a blue color, and a marker M5C is displayed by a one-dot chain line representing a white color. Since identifiability can be improved in a case where the colors of the markers are changed in this way, measurement can be easily performed.

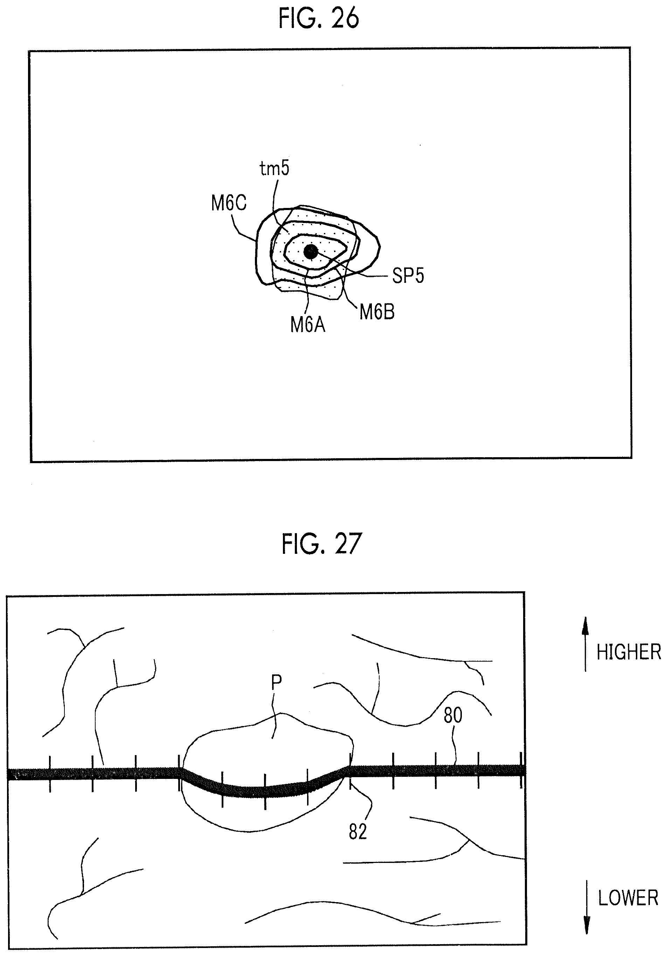

As shown in Fig. 26, a plurality of distorted concentric circular markers, which are distorted from the respective concentric circles, may be used as the first measurement marker other than the plurality of concentric circular markers. In this case, distorted concentric circular markers M6A, M6B, and M6C are displayed around a spot display portion SP5, which is formed on a tumor tm5, in the first captured image.

For auxiliary measurement light, light formed as a spot in a case of irradiating a subject light formed as a spot is used, but other light may be used. For example, as shown in Fig. 27, in the case of irradiating the subject, planar auxiliary measurement light formed as the crossing line 80 on the subject may be used. In this case, as the measurement marker, a second measurement marker including the crossing line 80 and the gradations 82 serving as an index of the size of the subject (for example, polyp P) on the crossing line is generated. In a case where planar auxiliary measurement light is used, the position specifying unit 50 specifies the position of the crossing line 80 (specific region). The lower the crossing line 80 is, the nearer the observation distance is, and the higher the crossing line 80 is, the farther the observation distance is. Therefore, as the crossing line 80 is positioned lower, the interval of the gradations 82 becomes larger, and as the crossing line 80 is positioned higher, the interval of the gradations 82 becomes smaller.

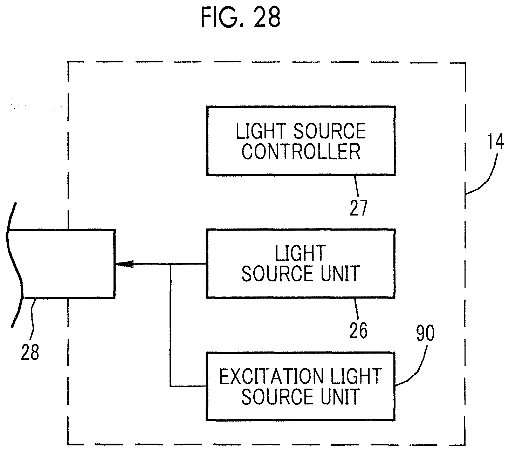

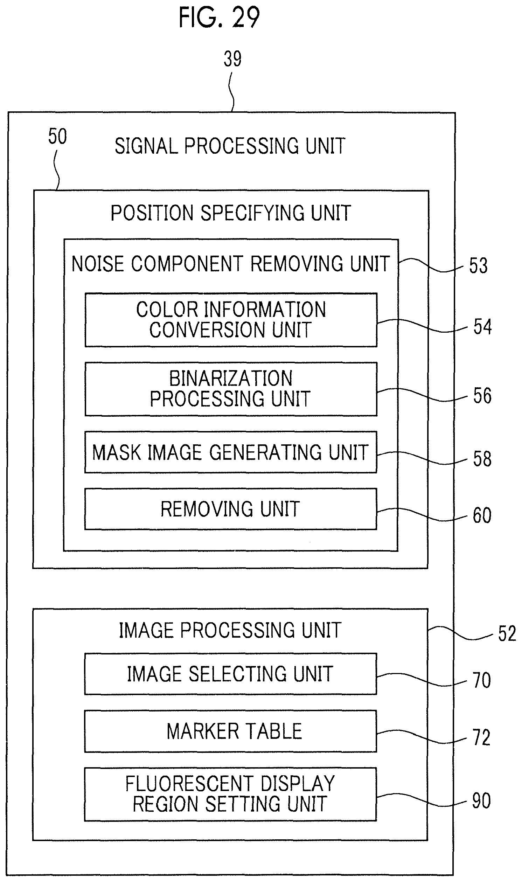

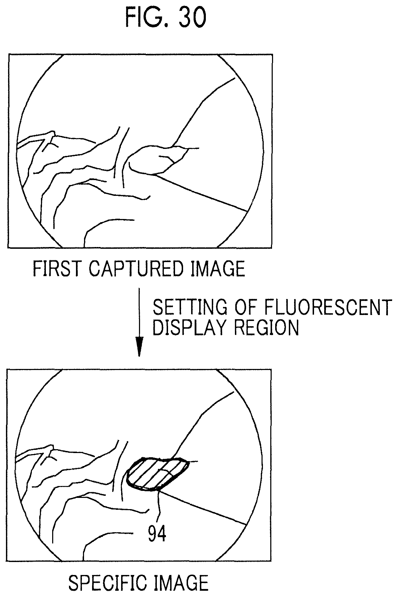

In the above embodiments, auxiliary measurement light is used as the specific light in order to display the measurement marker on the captured image, but the specific light may be used for other purposes. For example, excitation light is used as the specific light in order to excite and emit fluorescent components (auto-fluorescence, drug fluorescence) included in the subject. In this case, as shown in Fig. 28, an excitation light source unit 90 for emitting excitation light is provided in the light source device 14. Then, as shown in Fig 29, the position specifying unit 50 specifies the position of the region (specific region) of the fluorescent component from the second captured image obtained in the case where the excitation light is emitted together with the illumination light. In addition, a fluorescent display region setting unit 92 provided in the image processing unit 52 sets a fluorescent display region for displaying the fluorescent component according to the position of the fluorescent component region. Then, as shown in Fig. 30, a specific image is displayed on the monitor 18, where the fluorescent display region 94 is displayed on the first captured image obtained in a case where only the illumination light is emitted and the excitation light is not emitted. In this way, by displaying the region of the fluorescent component on the first captured image not including the excitation light component, it is possible to observe the region of the fluorescent component without impairing the color reproducibility of the subject.

In the embodiments, the hardware structures of processing units, which perform various kinds of processing, such as the signal processing unit 39, the display controller 40, and the system controller 41, are various processors to be described later. Various processors include: a central processing unit (CPU) that is a general-purpose processor functioning as various processing units by executing software (program); a programmable logic device (PLD) that is a processor of which the circuit configuration can be changed after the manufacture of a field programmable gate array (FPGA) and the like; a dedicated electrical circuit that is a processor having circuit configuration designed for exclusive use to perform various kinds of processing; and the like.

One processing unit may be formed of one of these various processors, or may be formed of a combination of two or more same kind or different kinds of processors (for example, a plurality of FPGAs or a combination of a CPU and an FPGA). Further, a plurality of processing units may be formed of one processor. As an example where a plurality of processing units are formed of one processor, first, there is an aspect where one processor is formed of a combination of one or more CPUs and software so as to be typified by a computer, such as a client or a server, and functions as a plurality of processing units. Second, there is an aspect where a processor fulfilling the functions of the entire system, which includes a plurality of processing units, by one integrated circuit (IC) chip is used so as to be typified by System On Chip (SoC) or the like. In this way, various processing units are formed using one or more of the above-mentioned various processors as hardware structures.

In addition, the hardware structures of these various processors are more specifically electrical circuitry where circuit elements, such as semiconductor elements, are combined.

### Explanation of References

10: endoscope apparatus
12: endoscope
12a: insertion part
12b: operation part
12c: bendable portion
12d: distal end portion
12e: angle knob
13a: mode changeover switch
13b: freeze switch
14: light source device
16: processor device
18: monitor
19: user interface
21: objective lens
22: illumination lens
23: auxiliary measurement lens
24: opening
25: air/water supply nozzle
26: light source unit
27: light source controller
28: light guide
29a: illumination optical system
29b: imaging optical system
30: auxiliary measurement light-emitting unit
30a: light source
30c: prism
32: imaging element
33: imaging controller
34: CDS/AGC circuit
36: communication interface (I/F)
37: still image storage unit
38: communication interface (I/F)
39: signal processing unit
40: display controller
41: system controller
50: position specifying unit
52: image processing unit
53: noise component removing unit
54: color information conversion unit
56: binarization processing unit
58: mask image generating unit
60: removing unit
62: color information on the noise component
63: noise component region
64: auxiliary measurement light color information
70: image selecting unit
72: marker table
80: crossing line
90: excitation light source unit
92: fluorescent display region setting unit
94: fluorescent display region
101: solid line
102: dotted line
EP: measurement point
Mx: gradation
M1, M2, M3: cruciform marker
tm1, tm2, tm3, tm4, tm5: tumor
SP: spot
SP1, SP2, SP3, SP4, SP5: spots
Lx1, Lx2: numbers of pixels in X direction
Ly1, Ly2: numbers of pixels in Y direction
M4A, M4B, M4C, M5A, M5B, M5C: concentric circular markers
M6A, M6B, M6C: distorted concentric circular markers
P: polyp

## Claims

1. An endoscope apparatus comprising:
an illumination light source unit that emits illumination light to illuminate a subject;
a specific light source unit that emits specific light;
a light emission controller that performs a control of lighting of the illumination light and performs a control of repeatedly lighting and dimming of the specific light at a specific frame interval;
an imaging element that images the subject;
an imaging acquisition unit that acquires a first captured image obtained by imaging the subject illuminated with the illumination light and a second captured image obtained by imaging the subject illuminated with the illumination light and the specific light;
a position specifying unit that specifies a position of a specific region to be obtained based on the specific light in the second captured image, based on the first captured image and the second captured image;
an image processing unit that processes the first captured image or the second captured image to generate a specific image, based on the position of the specific region; and
a display controller that causes a display unit to display the specific image.

2. The endoscope apparatus according to claim 1,
wherein the first captured image or the second captured image includes a noise component that interferes with the specifying of the position of the specific region by the position specifying unit, and
wherein the position specifying unit includes a noise component removing unit that removes the noise component from the second captured image to obtain a noise-removed second captured image based on the first captured image and the second captured image, and specifies the position of the specific region based on the noise-removed second captured image.

3. The endoscope apparatus according to claim 2,
wherein the noise component removing unit includes:
a color information conversion unit that converts the first captured image into a first color information image and converts the second captured image into a second color information image;
a binarization processing unit that binarizes the first color information image into a binarized first color information image and binarizes the second color information image into a binarized second color information image;
a mask image generating unit that obtains a mask image to remove the noise component from the second captured image based on the binarized first color information image and the binarized second color information image; and
a removing unit that obtains the noise-removed second captured image based on the binarized second color information image and the mask image.

4. The endoscope apparatus according to any one of claims 1 to 3,
wherein the image processing unit includes an image selecting unit that selects a processing target image which is a target to be processed from among the first captured image and the second captured image, and processes the image selected as the processing target image based on the position of the specific region.

5. The endoscope apparatus according to claim 4,
wherein the image selecting unit selects the processing target image based on a state relating to the position of the specific region.

6. The endoscope apparatus according to claim 4,
wherein the image selecting unit selects the processing target image according to an instruction from a user.

7. The endoscope apparatus according to any one of claims 1 to 6,
wherein the specific light is auxiliary measurement light used to measure the subject, and
wherein the image processing unit generates the specific image in which a measurement marker that is set according to the position of the specific region is displayed in a superimposed manner on the first captured image or the second captured image.

8. The endoscope apparatus according to claim 7,
wherein the measurement marker includes a first measurement marker indicating an actual size of the subject or a second measurement marker including a crossing line which is formed on the subject by the auxiliary measurement light and gradations serving as an index of a size of the subject on the crossing line.

9. The endoscope apparatus according to claim 7 or 8,
wherein a type of the measurement marker is selectable by an instruction from a user.

10. The endoscope apparatus according to any one of claims 1 to 6,
wherein the specific light is excitation light to cause a fluorescent component included in the subject to be excited and emitted, and
wherein the image processing unit sets a fluorescent display region to display the fluorescent component according to the position of the specific region to generate the specific image that displays the fluorescent display region on the first captured image.

## Patentansprüche

1. Endoskopvorrichtung, umfassend:
eine Beleuchtungslichtquelleneinheit, die Beleuchtungslicht zum Beleuchten eines Subjekts emittiert;
eine Spezifischlicht-Quelleneinheit, die spezifisches Licht emittiert;
eine Lichtemissionssteuerung, die eine Steuerung der Helligkeit des Beleuchtungslichts ausführt und eine Steuerung des wiederholten Erhellens und Dimmens des spezifischen Lichts bei einem spezifischen Einzelbildintervall ausführt;
ein Bildgebungselement, welches das Subjekt abbildet;
eine Abbildungserfassungseinheit, die ein erstes aufgenommenes Bild erfasst, gewonnen durch Abbilden des mit dem Beleuchtungslicht beleuchteten Subjekts, und ein zweites aufgenommenes Bild erfasst, erhalten durch Abbilden des mit dem Beleuchtungslicht und dem spezifischen Licht beleuchteten Subjekts;
eine Ortsspezifiziereinheit, die einen Ort einer spezifischen Zone spezifiziert, die zu erhalten ist auf der Grundlage des spezifischen Lichts in dem zweiten aufgenommenen Bild, basierend auf dem ersten aufgenommenen Bild und dem zweiten aufgenommenen Bild;
eine Bildverarbeitungseinheit, die das erste aufgenommene Bild oder das zweite aufgenommene Bild zum Erzeugen eines spezifischen Bilds verarbeitet, basierend auf dem Ort der spezifischen Zone; und
eine Anzeigesteuerung, die eine Anzeigeeinheit veranlasst, das spezifische Bild anzuzeigen.

2. Endoskopvorrichtung nach Anspruch 1,
bei der das erste aufgenommene Bild oder das zweite aufgenommene Bild eine Rauschkomponente enthält, die mit dem Spezifizieren des Orts der spezifischen Zone durch die Ortsspezifiziereinheit interferiert, und
wobei die Ortsspezifiziereinheit eine Rauschkomponenten-Entfernungseinheit enthält, die die Rauschkomponente aus dem zweiten aufgenommenen Bild entfernt, um ein rauschbereinigtes zweites aufgenommenes Bild zu erhalten, basierend auf dem ersten aufgenommenen Bild und dem zweiten aufgenommenen Bild, und die den Ort der spezifischen Zone basierend auf dem rauschbereinigten zweiten aufgenommenen Bild spezifiziert.

3. Endoskopvorrichtung nach Anspruch 2,
bei der die Rauschkomponenten-Entfernungseinheit enthält:
eine Farbinformations-Wandlereinheit, die das erste aufgenommene Bild in ein erstes Farbinformationsbild umwandelt und das zweite aufgenommene Bild in ein zweites Farbinformationsbild umwandelt;
eine Binärumsetzeinheit, die das erste Farbinformationsbild in ein binäres erstes Farbinformationsbild umsetzt und das zweite Farbinformationsbild in ein binäres zweites Farbinformationsbild umsetzt;
eine Maskenbilderzeugungseinheit, die ein Maskenbild zum Entfernen der Rauschkomponente aus dem zweiten aufgenommenen Bild basierend auf dem binär umgesetzten ersten Farbinformationsbild und dem binär umgesetzten zweiten Farbinformationsbild gewinnt; und
eine Entfernungseinheit, die das rauschbereinigte zweite aufgenommene Bild basierend auf dem binär umgesetzten zweiten Farbinformationsbild und dem Maskenbild gewinnt.

4. Endoskopvorrichtung nach einem der Ansprüche 1 bis 3, bei dem die Bildverarbeitungseinheit eine Bildauswahleinheit enthält, die ein VerarbeitungsZielbild, das ist ein zu verarbeitendes Ziel, aus dem ersten aufgenommenen Bild und dem zweiten aufgenommenen Bild auswählt und das ausgewählte Bild als Verarbeitungszielbild basierend auf dem Ort der spezifischen Zone verarbeitet.

5. Endoskopvorrichtung nach Anspruch 4,
bei der die Bildauswahleinheit das Verarbeitungszielbild basierend auf einem Zustand bezüglich des Orts der spezifischen Zone auswählt.

6. Endoskopvorrichtung nach Anspruch 4,
bei der die Bildauswahleinheit das Verareitungszielbild nach Maßgabe eines Benutzerbefehls auswählt.

7. Endoskopvorrichtung nach einem der Ansprüche 1 bis 6,
bei der das spezifische Licht ein Hilfsmesslicht zum Messen des Subjekts ist, und
wobei die Bildverarbeitungseinheit das spezifische Bild erzeugt, in welchem eine Messmarkierung, die nach Maßgabe des Orts der spezifischen Zone eingestellt ist, in Überlagerung mit dem ersten aufgenommenen Bild angezeigt wird.

8. Endoskopvorrichtung nach Anspruch 7,
bei der die Messmarkierung eine erste Messmarkierung enthält, die eine aktuelle Größe des Subjekts angibt, und eine zweite Messmarkierung enthält, die eine Kreuzungslinie enthält, gebildet auf dem Subjekt durch das Hilfsmesslicht und Gradationen, die als Index für eine Größe des Subjekts auf der Kreuzungslinie dient.

9. Endoskopvorrichtung nach Anspruch 7 oder 8,
bei der ein Typ der Messmarkierung durch einen Benutzerbefehl auswählbar ist.

10. Endoskopvorrichtung nach einem der Ansprüche 1 bis 6,
bei der das spezifische Licht ein Anregungslicht ist, um eine in dem Subjekt enthaltene fluoreszierende Komponente anzuregen und zur Emission zu veranlassen, und
wobei die Bildverarbeitungseinheit eine Fluoreszenzanzeigezone zum Anzeigen der Fluoreszenzkomponente nach Maßgabe des Orts der spezifischen Zone einstellt, um das spezifische Bild zu erzeugen, welches die fluoreszierende Anzeigezone an dem ersten Bild anzeigt.

## Revendications

1. Appareil d'endoscope, comprenant :
une unité de source de lumière d'éclairage, laquelle émet une lumière d'éclairage pour éclairer un sujet ;
une unité de source de lumière spécifique, laquelle émet une lumière spécifique ;
un contrôleur d'émission de lumière, lequel réalise une commande d'éclairage de la lumière d'éclairage, et réalise une commande répétée d'éclairage et de gradation de la lumière spécifique à un intervalle de trame spécifique ;
un élément d'imagerie, lequel image le sujet ;
une unité d'acquisition d'imagerie, laquelle acquiert une première image capturée obtenue en imageant le sujet éclairé avec la lumière d'éclairage et une seconde image capturée obtenue en imageant le sujet éclairé avec la lumière d'éclairage et la lumière spécifique ;
une unité de spécification de position, laquelle spécifie une position d'une région spécifique à obtenir sur la base de la lumière spécifique dans la seconde image capturée, sur la base de la première image capturée et de la seconde image capturée ;
une unité de traitement d'images, laquelle traite la première image capturée ou la seconde image capturée afin de générer une image spécifique, sur la base de la position de la région spécifique, et
un contrôleur d'affichage, lequel fait en sorte qu'une unité d'affichage affiche l'image spécifique.

2. Appareil d'endoscope selon la revendication 1,
dans lequel la première image capturée ou la seconde image capturée inclut une composante de bruit, laquelle perturbe la spécification de la position de la région spécifique par l'unité de spécification de position, et
dans lequel l'unité de spécification de position inclut une unité d'élimination de composante de bruit, laquelle élimine la composante de bruit de la seconde image capturée afin d'obtenir une seconde image capturée ayant subi une élimination de bruit sur la base de la première image capturée et de la seconde image capturée, et spécifie la position de la région spécifique sur la base de la seconde image capturée ayant subi une élimination de bruit.

3. Appareil d'endoscope selon la revendication 2,
dans lequel l'unité d'élimination de composante de bruit inclut :
une unité de conversion d'informations de couleur, laquelle convertit la première image capturée en une première image d'informations de couleur, et convertit la seconde image capturée en une seconde image d'informations de couleur ;
une unité de traitement de binarisation, laquelle binarise la première image d'informations de couleur en une première image d'informations de couleur binarisée, et binarise la seconde image d'informations de couleur en une seconde image d'informations de couleur binarisée,
une unité de génération d'image de masque, laquelle obtient une image de masque afin d'éliminer la composante de bruit de la seconde image capturée sur la base de la première image d'informations de couleur binarisée et de la seconde image d'informations de couleur binarisée, et
une unité d'élimination, laquelle obtient la seconde image capturée ayant subi une élimination de bruit sur la base de la seconde image d'informations de couleur binarisée et de l'image de masque.

4. Appareil d'endoscope selon l'une quelconque des revendications 1 à 3,
dans lequel l'unité de traitement d'images inclut une unité de sélection d'images, laquelle sélectionne une image cible de traitement, laquelle est une cible à traiter parmi la première image capturée et la seconde image capturée, et traite l'image sélectionnée comme l'image cible de traitement sur la base de la position de la région spécifique.

5. Appareil d'endoscope selon la revendication 4,
dans lequel l'unité de sélection d'images sélectionne l'image cible de traitement sur la base d'un état lié à la position de la région spécifique.

6. Appareil d'endoscope selon la revendication 4,
dans lequel l'unité de sélection d'images sélectionne l'image cible de traitement conformément à une instruction provenant d'un utilisateur.

7. Appareil d'endoscope selon l'une quelconque des revendications 1 à 6,
dans lequel la lumière spécifique est une lumière de mesure auxiliaire utilisée pour mesurer le sujet, et
dans lequel l'unité de traitement d'images génère l'image spécifique où un marqueur de mesure, lequel est réglé conformément à la position de la région spécifique, est affiché de manière superposée sur la première image capturée ou la seconde image capturée.

8. Appareil d'endoscope selon la revendication 7,
dans lequel le marqueur de mesure inclut un premier marqueur de mesure indiquant une taille réelle du sujet ou un second marqueur de mesure indiquant une ligne de croisement, laquelle est formée sur le sujet par la lumière de mesure auxiliaire et des gradations servant d'indice d'une taille du sujet sur la ligne de croisement.

9. Appareil d'endoscope selon la revendication 7 ou 8,
dans lequel un type du marqueur de mesure peut être sélectionné par une instruction provenant d'un utilisateur.

10. Appareil d'endoscope selon l'une quelconque des revendications 1 à 6,
dans lequel la lumière spécifique est une lumière d'excitation pour faire en sorte qu'une composante fluorescente incluse dans le sujet soit excitée et émise, et
dans lequel l'unité de traitement d'images règle une région d'affichage fluorescente afin d'afficher la composante fluorescente conformément à la position de la région spécifique, afin de générer l'image spécifique, laquelle affiche la région d'affichage fluorescente sur la première image capturée.
